Europäisches Patentamt

European Patent Office

Office européen des brevets

(11) Publication number: **0 162 302**
**B1**

# EUROPEAN PATENT SPECIFICATION

(45) Date of publication of patent specification: 06.12.89

(51) Int. Cl.⁴: **G 01 N 33/52**

(21) Application number: 85104792.8

(22) Date of filing: 19.04.85

(54) Integral multilayer analytical element.

(30) Priority: 19.04.84 JP 79158/84

(43) Date of publication of application:
27.11.85 Bulletin 85/48

(45) Publication of the grant of the patent:
06.12.89 Bulletin 89/49

(84) Designated Contracting States:
DE GB

(56) References cited:
EP-A-0 044 775
EP-A-0 097 952
EP-A-0 101 945
EP-A-0 103 901
GB-A-2 085 159

(73) Proprietor: FUJI PHOTO FILM CO., LTD.
210 Nakanuma Minami Ashigara-shi
Kanagawa 250-01 (JP)

(72) Inventor: Arai, Fuminori
c/o Fuji Photo Film Co., Ltd 3-11-46
Senzui Asaka-shi Saitama (JP)
Inventor: Yazawa, Kenichiro
c/o Fuji Photo Film Co., Ltd 3-11-46
Senzui Asaka-shi Saitama (JP)
Inventor: Katsuyama, Harumi
c/o Fuji Photo Film Co., Ltd 3-11-46
Senzui Asaka-shi Saitama (JP)
Inventor: Osaka, Chiaki
c/o Fuji Photo Film Co., Ltd 3-11-46
Senzui Asaka-shi Saitama (JP)

(74) Representative: Kraus, Walter, Dr. et al
Patentanwälte Kraus, Weisert & Partner
Thomas-Wimmer-Ring 15
D-8000 München 22 (DE)

Courier Press, Leamington Spa, England.

**Description**

This invention relates to an integral multilayer analytical element for the analysis of an analyte in a liquid sample having, in order, a porous spreading layer, a water-absorbing or water-permeable layer containing a hydrophilic polymer which may contain at least one reagent being reactive to the analyte in the liquid sample to show a detectable change, and a light-transmissive, water-impermeable support. More particularly, this invention relates to an integral multilayer analytical element advantageously employable in an analysis of an aqueous liquid sample, especially in a clinical test using a body fluid as a sample.

Until now, as dry-type analytical elements there have been proposed a number of integral multilayer analytical elements (hereinafter occasionally referred to simply as multilayer analytical elements) which comprise a transparent support, a water-absorbing reagent layer containing a hydrophilic polymer (binder) and a color-forming reagent superposed on the support and a porous spreading layer (hereinafter occasionally referred to simply as spreading layer) as the uppermost layer. The spreading layer of the multilayer analytical element functions to spread therein an aqueous liquid sample (e.g., blood such as whole blood, plasma or serum, lymph, saliva, spinal fluid, vaginal fluid, urine, drinking water, liquers, river water and water discharged from factories) which has been applied onto the surface of the spreading layer together with the components contained in the liquid sample in a lateral direction to supply the liquid sample into the water-absorbing layer (or a water-absorbing reagent-containing layer containing a hydrophilic polymer) at a uniform volume per unit surface area. This function is named a metering effect. As the porous spreading layers, there have been proposed non-fibrous anisotropic microporous medium layer, typically a membrane filter disclosed in JP—A—49(1974)-53888 (Patent Publication No. 53(1978)-21677) and US—A—3,992,158, three-dimensional lattice structure layer having continuous voids in which polymer microbeads are combined with each other through an adhesive agent resistant to swell with water disclosed in JP—A—55(1980)-90859 (US—A—4,258,001), spreading layers of woven fabric layer disclosed in JP—A—55(1980)-164356 (US—A—4,292,272) and 57(1982)-66359; and a spreading layer of paper sheet comprising fibrous pulps of hydrophobic polymer such as polyethylene. Among these spreading layers, the spreading layer of woven fabric is capable of spreading satisfactorily all of a whole blood, a plasma, and a serum. Further, the spreading layer of woven fabric is very advantageous from the viewpoint of the easy handling for manufacture of an integral multilayer analytical element, as well as from the viewpoint of the superior mechanical strength of the element.

EP—A—0 097 952 describes a multilayer analytical element for fluorometric assay of an analyte contained in an aqueous sample liquid, utilizing a competitive binding reaction to a protein between the analyte and a fluorescent-labelled analyte substance or its analogue. The reaction involved is an immunological reaction. The analytical element comprises a porous spreading sheet (101), a porous sharing sheet (111) and a reaction sheet (121). The term of "sheet" is used for the layer in EP—A—0 097 952. The porous sharing sheet functions for sharing the free fluorescent-labelled substance (F) into other space than the space of the reaction sheet. For facilitating the function of sharing the liquid, it is not preferable to combine these sheets, for instance using an adhesive, to form an integral structure. Thus, the term of "sheet" is employed in EP—A—0 097 952 to mean that each functional portion is prepared independently and then arranged by simply overlaying one on another. Accordingly, the spreading layer of EP—A—0 097 952 is not the same as the spreading layer of the integral multilayer analytical element as defined above which has an integral structure comprising the spreading layer and the hydrophilic polymer-containing water-absorbing or water-permeable layer arranged under the spreading layer.

It has been now found, however, that the spreading layer of woven fabric (hereinafter referred to as "woven fabric spreading layer") is hardly adjustable to vary the spread area (or spreadable area) from the inherently-given spread area, maintaining the necessary metering effect. For instance, if an analyte content in an aqueous liquid sample (hereinafter occasionally referred to as simply "a liquid sample" or "test liquid") is small, it is desirable to decrease the spread area (i.e., area in which the liquid sample is spread). In the case that the woven fabric spreading layer is employed as the spreading layer, the decrease of the spread area is difficultly attained or is only attained by reducing the metering action.

The object of the present invention is to provide an integral multilayer analytical element having a porous spreading layer which is made of a new material and shows a spreading action (i.e., metering action) similar to the known woven facric, the spread area of which is easily adjustable over a wide range without reducing the spreading action (metering action).

When employing blood as the aqueous liquid sample the use of said element should facilitate determination of a relationship between a value measured on a whole blood sample and a value measured on a plasma or serum sample and further conversion between the measured values should be facilitated.

Finally, it should become possible to determine a hematocrit value and a hemoglobin concentration in a whole blood sample.

The present invention provides an integral multilayer analytical element as described above which is characterized in that said porous spreading layer is made of knitted fabric capable of spreading the liquid sample applied thereto in a lateral direction so as to supply the liquid sample into the water-absorbing or water-permeable layer, at a substantially uniform volume per unit surface area.

Thus, the principal characteristic feature of the present invention resides in the use of a spreading layer of knitted fabric.

As the light-transmissive, water-permeable support of the integral multilayer analytical element of the invention, there can be mentioned light-transmissive, water-impermeable supports employed in the multilayer analytical elements disclosed in the aforementioned patent publications. A representative support is a transparent polymer support having a thickness of approx. 50 $\mu$m to approx. 1 mm, preferably approx. 80 $\mu$m to approx. 300 $\mu$m. Examples of the polymer include polyethylene terephthalate, polycarbonate of bisphenol A, polystyrene, and cellulose esters (e.g., cellulose diacetate, cellulose triacetate, cellulose acetate propionate). If necessary, a subbing layer or an adhesive layer (which is known as such by the aforementioned patent publications) can be provided to the surface of support so as to strengthen the bonding between the support and the below-mentioned water-absorbing layer or reagent-containing layer.

The water-absorbing layer comprises as a main component a hydrophilic polymer which swells upon absorbing water, and accordingly is capable of absorbing a water reaching the surface thereof or permeating therein. The hydrophilic polymer can be chosen from natural or synthetic hydrophilic polymer having a swelling index of approx. 150 to 2,000%, preferably approx. 250 to 1,500% (water at 30°C). Examples of the hydrophilic polymer include gelatins (e.g., acid-treated gelatin, deionized gelatin, etc.) gelatin derivatives (e.g., phthalated gelatin, hydroxyacrylate-grafted gelatin, etc.) agarose, pullulan, pullulan derivatives, polyacrylamide, polyvinyl alcohol, and polyvinylpyrrolidone. The water-absorbing layer generally has a thickness of approx. 1 to 100 $\mu$m, preferably approx. 3 to 50 $\mu$m, most preferably approx. 5 to 30 $\mu$m, under dry condition. Preferably, the water-absorbing layer is transparent. The water-absorbing layer can contain a known mordant, basic polymer and/or acidic polymer.

The water-absorbing or water-permeable reagent-containing layer (occasionally referred to simply as "reagent layer") is a substantially nonporous water-absorbing layer or a microporous water permeable layer which contains a hydrophilic polymer (binder) and at least one reagent producing a detectable change upon reaction with an analyte (a component to be determined) in a liquid sample. The detectable change generally means a change which is detectable by a photometric measurement. Examples of the detectable change include color change, color formation, emission of fluorescence, change of absorbance in the ultraviolet region, and production of turbidity.

The reagent to be incorporated into the reagent layer is chosen according to the combination of an analyte in a liquid sample and a reaction system chosen for performing the analysis of the analyte. In the case that two or more reagents participate in the reaction, these reagents can be incorporated into one reagent layer, for instance, in the form of a mixture, and otherwise these reagents can be incorporated independently into plural layers. Examples of the reagent to be incorporated into the reagent layer include reagents containing enzyme(s) disclosed in the aforementioned patent publications, other known analytical reagent(s) and reagents employed for biochemical tests in clinical diagnosis.

The hydrophilic polymer employed for the formation of the substantially nonporous water-absorbing reagent layer serves as a medium for substantially uniformly dissolving or dispersing therein the reagent or reagent composition. Moreover, the hydrophilic polymer serves for absorbing the water of the liquid sample so as to supply the analyte together with water into the reagent layer. The hydrophilic polymer employed for this purpose can be selected from the water-absorbing hydrophilic polymers described hereinbefore with respect to the water-absorbing layer. The substantially non-porous reatent layer generally has a thickness of approx. 3 to 50 $\mu$m, preferably approx. 5 to 30 $\mu$m, under dry condition. Preferably, the reagent layer is transparent.

The microporous water-permeable reagent layer is a microporous layer containing a reagent or reagent composition within a microporous matrix layer formed by a solid particulate and a hydrophilic polymer (binder). In more detail, the microporous matrix layer is formed by binding microporous particles or nonporous particles with the hydrophilic polymer binder to give a microporous continuous void structure.

Examples of the microporous particles and nonporous particles employed for the formation of the microporous reagent layer include cellulose microparticles such as microcrystalline cellulose, cellulose micropowder, and cellulose microparticles; silicon dioxide microparticles such as silica and diatomaceous earth; polymer beads, glass beads and ceramic beads. The hydrophilic polymer can be chosen from the water-absorbing hydrophilic polymer described hereinbefore with respect to the water absorbing layer. Also employable in an aqueous latex of a copolymer containing a hydrophilic group as a repeating constitutional unit in an amount of not less than approx. 2%, as disclosed in Japanese Patent Provisional Publication No. 59(1984)-145965. The microporous reagent layer generally has a thickness of approx. 7 to 50 $\mu$m, preferably approx. 10 to 30 $\mu$m, under dry condition.

The reagent layer can contain a known pH buffer composition, a polymer pH buffer, a basic polymer, an acidic polymer, a polymer mordant, etc.

The water-absorbing layer or the substantially non-porous reagent layer can be formed on the aforementioned light-transmissive, water-impermeable support according to a known coating method. The microporous reagent layer can be formed on the support according to the method disclosed in Japanese Patent Application No. 57(1982)-227980 or Japanese Patent Provisional Publication No. 59(1984)-145965. If necessary, the surface of the support can be processed according to the known physico-chemical or chemical activation method, or provided with a transparent subbing layer (e.g., gelatin subbing layer), so that the bonding between the support and the water-absorbing layer or between the support and the reagent layer can be strengthened. Moreover, a water-absorbing layer can be provided between the support and the reagent layer. Particularly, the provision of a water-absorbing layer between the support and the reagent layer is preferred in the case that the reagent layer is a microporous reagent layer.

On the reagent layer or water-absorbing layer, a light-shielding layer can be formed. The light-shielding layer is a water-passable or water-permeable layer in which light-shielding microparticles or light-shielding and light-reflective microparticles are dispersed in a small amount of a film-forming hydrophilic polymer. The light-shielding layer shields a color of an aqueous liquid sample, particularly the red color of hemoglobin contained in a whole blood sample, applied to the below-described porous spreading layer in the reflection measurement of the detectable change taking place in the reagent layer or water-absorbing layer on the light-transmissive support side. Further, the light-shielding layer can function as a light-reflecting layer and a background layer.

Examples of the light-shielding and light-reflecting particles include titanium dioxide microparticles (e.g., titanium dioxide microcrystalline particles of the rutile type, anatase type or brookite type, having particle size of approx. 0.1 to 1.2 µm), barium sulfate microparticles, and aluminum microparticles or micro-flakes. Examples of the light-shielding microparticles include carbon black, gas black and carbon microbeads. Among these microparticles, titanium dioxide microparticles and barium sulfate microparticles are preferred. As the film-forming hydrophilic polymer, there can be mentioned the hydropholic polymer stated hereinbefore with respect to the water-absorbing layer, and weak water-hydrophilic regenerated cellulose and cellulose acetate. Among these polymers, gelatin, gelatin derivatives, and polyacrylamide are preferred. The gelatin and gelatin derivatives can be employed in combination with a known hardening agent (i.e., cross-linking agent).

The light-shielding layer can be formed on the reagent layer or water-absorbing layer by coating an aqueous dispersion containing light-shielding microparticles and a hydrophilic polymer and then drying the coated layer in a known manner. The volume ratio of the hydrophilic polymer to the light-shielding microparticles under dry condition ranges generally from approx. 2.5 to 7.5, preferably from approx. 3.0 to 6.5, to 10 of the microparticles. In the case that the light-shielding microparticles are titanium dioxide microparticles, the hydrophilic polymer is incorporated in a weight ratio of generally approx. 0.6 to 1.8, preferably approx. 0.8 to 1.5, to 10 of the titanium dioxide. The light-shielding layer has a thickness of approx. 3 to 30 µm, preferably approx. 5 to 20 µm, under dry condition.

On the reagent layer, water-absorbing layer or light-shielding layer, an adhesive layer can be provided to assist the provision of the below-mentioned porous spreading layer. Especially in the case that the light-shielding layer is arranged, an adhesive layer is preferably incorporated. The adhesive layer can be composed mainly of a hydrophilic polymer which can be united with the porous spreading layer under such conditions that the adhesive layer is wetted or swollen with water. As the hydrophilic polymer, there can be mentioned the hydrophilic polymers described hereinbefore with respect to the water-absorbing layer. Among these hydrophilic polymers, gelatin, gelatin derivatives and polyacrylamide are preferred. The adhesive layer has a thickness of approx. 0.5 to 20 µm, preferably approx. 1 to 10 µm, under dry condition. The adhesive layer can contain a surfactant. The surfactant preferably is a nonionic surfactant, especially a nonionic surfactant having a chain structure of 8 to 15 oxyethylene or oxypropylene groups.

The adhesive layer can be formed on the reagent layer, water-absorbing layer or light-shielding layer by coating an aqueous solution containing a hydrophilic polymer and an optionally added surfactant in a known manner.

On the water-absorbing layer or reagent layer is provided a porous spreading layer of knitted fabric (or knitted fabric-like material) directly or via a light-shielding layer (or light-reflecting layer) and an adhesive layer. The knitted fabric spreading layer is capable of spreading laterally therein an aqueous liquid sample which has been applied onto the upper surface thereof (surface on the side farthermost from the light-transmissive support) to supply the components of the liquid sample into the water-absorbing layer or water-absorbing or water-permeable reagent layer (containing a hydrophilic polymer) at uniform volume per unit surface area, that is, a spreading or metering action. The knitted fabric spreading layer also functions as a filter layer to remove insoluble material in an aqueous liquid sample (e.g., red blood cell in a whole blood sample) which is apt to disturb the analysis.

As the knitted fabric (including knitted fabric-like material), there can be mentioned a wide variety of knitted materials such as warp knitting and weft knitting. Examples of the warp knitting include single atlas stitch fabric, single tricot stitch fabric, double tricot stitch fabric, milanese stitch fabric and raschel stitch fabric. Examples of the weft knitting include plain stitch fabric, pearl stitch fabric, rib stitch fabric and interlock stitch fabric. The knitted fabric is generally made of natural yarns such as cotton yarn, silk yarn and wool yarn; and yarns (including filaments) of regenerated cellulose such as viscous rayon and cuprammonium rayon, semisynthetic organic polymers such as cellulose diacetate and cellulose triacetate, and synthetic organic polymers such as polyamide (e.g., various nylon), acetylized polyvinyl alcohol (e.g., vinylon), polyacrylnitrile, polyethylene terephthalate, polyethylene, polypropylene and polyurethane, and mixtures of natural yarns and regenerated yarns, semisynthetic organic polymer yarns and synthetic organic polymer yarns. The yarn can be a filament yarn or spun yarn. The spun yarn is preferred. The yarn of the knitted fabric generally is in the range of approx. 40 S to 150 S (in terms of cotton spun yarn count),

4

preferably approx. 60 S to 120 S, or in the range of approx. 35 D to 130 D (in terms of silk yarn denier), preferably approx. 34 D to 90 D. The gauge number adopted for manufacturing the knitted fabric generally ranges from approx. 20 to approx. 50. The thickness of the knitted fabric ranges generally from approx. 100 µm to approx. 600 µm, preferably approx. 150 µm to approx. 400 µm. The void ratio of the knitted fabric ranges generally from approx. 40% to approx. 90%, preferably from approx. 50% to approx. 85%. Among the warp knittings, single tricot stitch fabric, double tricot stitch fabric, milanese stitch fabric and raschel stitch fabric are preferred, because these are hardly stretchable in the warp direction, are easily handled in the below-described lamination process of the knitted fabric spreading layer, and hardly become unlaced.

The knitted fabric to be employed for the formation of the knitted fabric spreading layer is substantially free from oil and fat which is apt to attach or supplied to the fabric in the course of the yarn manufacture or fabric manufacture and removed by a defatting (deoiling) process such as washing with water. The knitted fabric can be made hydrophilic, for instance, by a physical activation treatment (preferably, glow-discharge treatment or corona-dicharge treatment) applied to at least one side of the knitted fabric as disclosed in Japanese Patent Provisional Publication No. 57 (1982)-66359; or by a surfactant impregnation treatment (preferably, a non-ionic surfactant impregnation treatment), or a hydrophilic polymer impregnation treatment as disclosed in Japanese Patent Provisional Publications No. 55(1980)-164356 and No. 57 (1982)-66359; or an optional combination of two or more of these treatments. Thus treated hydrophilic knitted fabric is satisfactory in the adhesion to the underlayer or is adjustable or controllable in the spreadable area.

The knitted fabric can be laminated under adhesion on the reagent layer, water-absorbing layer or adhesive layer in the manner as disclosed in Japanese Patent Provisional Publications No. 55 (1980)-164356 and No. 57 (1982)-66359, etc. In more detail, a knitted fabric is applied under substantially uniform light pressure to the undried reagent layer, water-absorbing layer or adhesive layer which is provided just after the coating procedure is complete. Alternatively, the dried reagent layer, water-absorbing layer or adhesive layer is swollen by supplying water substantially uniformly into the layer and the knitted fabric is then applied onto the swollen layer in the same manner as above. Thus, the knitted fabric is combined with other layers to give the integral structure. In the case of employing gelatin or a gelatin derivative as the hydrophilic polymer binder in the reagent layer, water-absorbing layer or adhesive layer, the knitted fabric can be applied onto the undried gelatin (or gelatin derivative) layer still under a gelation condition just after the coating is complete, so as to give the desired integral structure.

The reagent composition or a portion or component thereof can be incorporated into the knitted fabric spreading layer. Especially in the case that the reagent composition includes a polymer substance, typically enzyme, or a dissociating agent for analyte conjugated with protein, said reagent component is almost preferably incorporated into the knitted fabric spreading layer. The incorporation of the reagent composition or a portion or component thereof into the spreading layer can be done in the manner as disclosed in Research Disclosure #12626 (October 1974), Japanese Patent Provisional Publication No. 50 (1975)-137192 (corresponding to US—A—3,983,002) and Japanese Patent Provisional Publication No. 57 (1982)-208997 (corresponding to US—A—4,452,887), for enzyme; Japanese Patent Provisional Publication No. 57 (1982)-208998, for substrate of enzyme; Japanese Patent Provisional Publication No. 59 (1984)-171864, for a dissociating agent for analyte associated with protein; Japanese Patent Provisional Publication No. 59 (1984)-49854 (Example 7), for other components.

The integral multilayer analytical element of the present invention can be provided, if necessary, with a detection layer or a mordant layer as disclosed in Japanese Patent Provisional Publication No. 51 (1976)-40191 (corresponding to US—A—4,042,335), Japanese Patent Provisional Publication No. 53 (1978)-131089 (corresponding to US—A—4,144,306); a migration-inhibition layer as disclosed in Japanese Patent Provisional Publication No. 54 (1979)-29700 (corresponding to US—A—4,166,093); an intermediate layer as disclosed in Japanese Patent Provisional Publication No. 51 (1976)-40191; a reagent-containing microparticle-dispersing layer as disclosed in Japanese Patent Provisional Publication No. 56 (1981)-8549 (corresponding to US—A—4,356,149); a uniform thin barrier layer of a specific hydrophobic polymer as disclosed in Japanese Patent Provisional Publication No. 52 (1977)-3488 (corresponding to US—E—30,267); an air barrier layer disclosed in Japanese Patent Provisional Publication No. 58 (1983)-77661 and Japanese Patent Application No. 58 (1983)-153822; and a gas-permeable adhesive intermediate layer as disclosed in Japanese Patent Application No. 58 (1983)-128759.

The integral multilayer analytical element of the present invention can be cut to give a small square chip (length of one side: approx. 15 to 30 mm) or round sheet of almost same size and encased in a slide frame, to give an analytical slide as disclosed in Japanese Patent Provisional Publication No. 57 (1982)-63452, Japanese Patent Provisional Publication No. 54 (1979)-156079 (corresponding to US—A—4,169,751), Japanese Utility Model Provisional Publication No. 56 (1981)-142454 (corresponding to US—A—4,387,990), Japanese Utility Model Provisional Publication No. 58 (1983)-32350, and Japanese Patent Provisional PCT Publication No. 58 (1983)-501144 (corresponding to WO 83/00391). The analytical element in the form of the analytical slide is advantageous in all aspects such as manufacture, packing, transfer, storage and measurement procedure.

The integral multilayer analytical element of the invention can be used in the manner as disclosed in the aforementioned patent publications. For instance, an aqueous liquid sample in the amount of approx. 5 to 30 µl, preferably approx. 8 to 15 µl, is spotted on the knitted fabric spreading layer, and if necessary (particularly in the case of employing an enzyme as a component of the reagent composition) the analytical element is then subjected to incubation at a substantially constant temperature in the range of 20 to 45°C. The element was photometrically measured through reflection photometry on the light-transmissive support side to detect a detectable change such as color change or color formation occurring in the element. The measured value was colorimetrically treated to quantitatively determine the analyte in the liquid sample. In the case of using an integral multilayer analytical element having a water-absorbing layer and a knitted fabric spreading layer on a light-transmissive, water-impermeable support, a hematocrit value or hemoglobin concentration in a blood can be determined in the same manner as in the case of using an integral multilayer analytical element as disclosed in Japanese Patent Provisional Publication No. 56 (1981)-96245 (US—A—4,340,565) and Japanese Patent Provisional Publication No. 56 (1981)-97872 (US—A—4,337,222).

The integral multilayer analytical element of the present invention can be prepared in the form of having a knitted fabric porous layer for supplying an aqueous liquid sample via a fixed area defined by a certain shape thereof (i.e., patch) which is provided as the uppermost layer, as disclosed in Japanese Utility Model Provisional Publication No. 57 (1982)-42951.

The present invention is further illustrated by the following examples and comparison examples.

Example 1

An aqueous gelatin solution having the below-stated composition was coated on a gelatin subbing layer of a colorless, transparent polyethylene terephthalate (PET) film (support, thickness: 180 μm) to give a layer of thickness of 20 μm (dry basis). The coated layer was then dried and hardened at 45°C for 3 days to give a water-absorbing layer.

Aqueous gelatin solution for forming water-absorbing layer

| | |
|---|---|
| Alkali-treated deionized gelatin | 200 g |
| Polyoxyethylene nonylphenyl ether (containing 10 (average) oxyethylene units) | 2 g |
| Bis(vinylsulfonylmethyl)ether | 1 g |

On the water-absorbing layer was coated an aqueous gelatin solution containing the polyoxyethylene nonylphenyl ether. The coated layer was dried to give an adhesive layer containing 0.2% of the nonionic surfactant (thickness: 3 μm, dry basis).

One surface of a tricot knitted fabric (thickness: approx. 250 μm) of polyethylene terephthalate (PET) spun yarn (count: equivalent to approx. 50 D) having been defatted by washing with water was processed by glow discharge for 60 sec. (1.6 kW/m$^2$, oxygen concentration was controlled under reduced pressure).

On the adhesive layer having been almost uniformly wetted with water was placed the glow discharge-processed tricot knitted fabric to face the processed surface with the adhesive layer. The composite was then passed between pressure rollers to uniformly combine the tricot knitted fabric with the ahdesive layer. Thus, on integral multilayer analytical element having a knitted fabric spreading layer was prepared.

On the spreading layer of some of the obtained multilayer analytical element was coated an aqueous solution of the following composition, varying the coating amount per unit area as shown in Table 1. The coated layer was then dried to prepare an integral multilayer analytical element containing a knitted fabric spreading layer showing a spreading action-controlled spreading action.

Coating solution for spreading action-controlling layer

| | |
|---|---|
| Hydroxypropylcellulose (methoxy group content: 28—30%, hydroxypropoxy group content: 7—12%, viscosity: 50 mPa · s (cps) at 20°C in 2% aqueous solution) | 2 g |
| Polyoxyethylene octylphenyl ether (containing 15(average) oxyethylene units) | 1 g |
| Water | 95 g |

On the knitted fabric-spreading layer of thus produced integral multilayer analytical element was spotted 10 μl of 7% aqueous human albumin solution. The spread area was measured. The results are set forth in Table 1

Comparison Example 1

The procedures of Example 1 were repeated except that the tricot knitted fabric was replaced with a plain weave fabric of the PET spun yarn (count: approx. 50 D). Thus, integral multilayer analytical elements of two kinds (i.e., simple type and spreading action-controlled type) were produced. The spread area was measured in the same manner as in Example 1. The results are set forth in Table 1.

TABLE 1

| Coated amount*1 (ml/m²) | Example 1 spread area (cm²) | Com. Ex. 1 spread area (cm²) |
|---|---|---|
| Non-coated | 1.95 | 3.23 |
| 130 | 1.52 | 1.74 |
| 160 | 1.45 | 1.50 |
| 180 | 1.38 | 1.28*2 |
| 220 | 1.35 | Impossible*3 |

Remarks

*1: Coated amount of the spreading action-controlling coating solution

*2: Spreading action was not uniform, and no smooth spreading action was given

*3: The spreading action-controlling coating solution was not uniformly coated on the fabric spreading layer.

The spreading action was satisfactory except in one comparison test run indicated in the above remarks. The results teach that the knitted fabric spreading layer introduced into the integral multilayer analytical element of the invention give satisfactory spreading action. Further, it is apparent that the knitted fabric spreading layer can be optionally controlled in the spreading action in a wide range without loosing the metering effect.

Example 2

On the integral multilayer analytical element of Example 1 having a knitted fabric spreading layer coated with the spreading action-controlling coating solution in the amount of 180 ml/m² was spotted water or 7% aqueous human albumin solution. The spread area was then measured. The results are set forth in Table 2.

Comparison Example 2

A broad cloth of 80 S cotton count (thickness: approx. 200 μm) was impregnated with aqueous solution containing 0.5% of polyoxyethylene nonylphenyl ether (having 10(average) oxyethylene units) and dried. Thus, the broad cloth was made hydrophilic. The broad cloth was laminated on the water-absorbing layer under pressure in the same manner as in Example 1 to give an integral multilayer analytical element without coating the spreading layer with a spreading action-controlling coating solution. The measurement of spread area was performed on thus produced multilayer analytical element in the same manner as in Example 2. The results are set forth in Table 2.

TABLE 2

| Spotted liquid | Example 2 spread area (cm²) | Com. Ex. 2 spread area (cm²) |
|---|---|---|
| Water | 1.29 | 1.62 |
| 7% aqueous albumin | 1.38 | 1.37 |

The spreading action was satisfactory at almost same level for the multilayer analytical elements of Example 2 and Comparison Example 2. However, the results given in Table 2 teach that the knitted fabric-spreading layer shows little difference of spread area regardless of change of the nature of spotted sample.

Example 3 and Comparison Example 3

The reagent layer for glucose determination (thickness: approx. 15 μm, dry basis) was formed on a gelatin subbing layer of a colorless, transparent PET film (thickness: 185 μm) using the following composition.

Reagent composition for glucose determination

| | |
|---|---|
| Glucode oxidase | 15,000 IU |
| Peroxidase | 25,000 IU |
| 1,7-Dihydroxynaphthalene | 5 g |
| 4-Aminoantipyrine | 12 g |
| Deionized gelatin | 200 g |
| Polyoxyethylene nonylphenyl ether (containing 10(average) oxyethylene units) | 2 g |

On the reagent layer was coated an aqueous dispersion containing 0.2 g of polyoxyethylene nonylphenyl ether (containing 10(average) oxyethylene units), 8 g of titanium dioxide microparticles (rutile type, particle size: 0.25—0.40 µm), and 1 g of deionized gelatin. The coated layer was dried to form a light-shielding layer of approx. 5 µm thick (dry basis).

On the light-shielding layer was coated a solution of 4 g of deionized gelatin and 0.2 g of polyoxyethylene nonylphenyl ether (containing 10(average) oxyethylene units) in 100 ml of water. The coated layer was dried to form an adhesive layer of approx. 2 µm thick (dry basis).

On the adhesive layer was provided a porous spreading layer of tricot knitted fabric (thickness: 250 µm) of PET spun yarn (count: equivalent to 50 D) in the same manner as in Example 1. On the spreading layer was coated the spreading action-controlling coating solution having the below-stated composition in the amount of 150 ml per 1 m². The coated layer was dried to give an integral multilayer analytical element for quantitative determination of glucose concentration in blood (Example 3).

Spreading action-controlling coating solution

| | |
|---|---|
| Methylcellulose (viscosity 100 cps at 20°C, measured on 2% aqueous solution) | 20 g |
| Titanium dioxide microparticles (rutile type, particle size: 0.25—0.40 µm) | 100 g |
| Polyoxyethylene octylphenyl ether (containing 30(average) oxyethylene units) | 10 g |
| Water | 1000 g |

Separately, an integral multilayer analytical element for quantitative determination of glucose concentration in blood was prepared in the same manner as above (Example 3) except that the porous spreading layer was produced using a plain cloth of cotton yarn of 100 D count which had not subjected to glow discharge treatment and was not coated with the spreading action-controlling coating solution (Comparison Example 3).

On each of the integral multilayer analytical elements for quantitative determination of glucose in blood (the elements of Example 3 and Comparison Example 3) was spotted 10 µl of a human serum having the glucose content indicated in Table 3 which was prepared by adding glucose to a human serum. The element was subjected to incubation at 37°C for 6 min. The color formation shown on the element was then measured by reflection photometry at a visible ray of 500 nm (central wavelength) using a reflection optical densitometer. The measured values are set forth in Table 3.

TABLE 3

| Glucose concentration (mg/dl) | Measured reflection optical density | |
|---|---|---|
| | Example 3 | Com. Ex. 3 |
| 98 | 0.621 | 0.565 |
| 205 | 0.904 | 0.837 |
| 350 | 1.192 | 1.061 |
| 495 | 1.429 | 1.228 |
| 603 | 1.561 | 1.304 |
| Diameter of spread circle | 13 mm | 12 mm |

Remark

The glucose concentration value of the human serum was obtained by Hexokinase-G-6-PDH method.

An aqueous solution at a glucose concentration of 100 mg/dl (value determined according to Hexokinase-G-6-PDH method) containing a protein (human albumin) in the amount indicated in Table 4 was prepared. The aqueous solution was spotted on the porous spreading layer of the integral multilayer analytical elements (elements of Example 3 and Comparison Example 3) in the amount of 10 μl. The element was subjected to incubation at 37°C for 6 min. Immediately after the incubation, the color formation was measured by reflection photometry at 500 nm (central wavelength) on the support side. The glucose concentration was calculated from the optical density using a calibration curve.

TABLE 4

| | Glucose concentration mg/dl (measured value) | |
|---|---|---|
| Protein (g/dl) | Example 3 | Com. Ex. 3 |
| 4 | 98 | 93 |
| 7 | 100 | 100 |
| 10 | 101 | 108 |

The results set forth in Table 4 indicate that the integral multilayer analytical element of the invention having the knitted fabric spreading layer gives a calibration curve having an incline larger than that given by the conventional integral multilayer analytical element having a woven fabric spreading layer. Further, the integral multilayer analytical element of the invention gives a calibration curve having a large incline even in the region of high glucose concentration, and the measured value hardly varies with the content of protein in an aqueous liquid sample. For the reasons, the analytical element of the invention makes it possible to determine very accurately the glucose content over wide range.

Example 4 and Comparison Example 4

An aqueous gelatin solution having the below-stated composition was coated on a gelatin subbing layer of a colorless, transparent polyethylene terephthalate (PET) film support, thickness: 180 μm) to give a layer of thickness of 20 μm (dry basis). The coated layer was then dried to give a water-absorbing layer.

Aqueous gelatin solution for forming water-absorbing layer

| | |
|---|---|
| Deionized gelatin | 10 g |
| Polyoxyethylene nonylphenyl ether (containing 10(average) oxyethylene units) | 0.01 g |
| Water | 80 ml |

On the water-absorbing layer was provided a spreading layer of a tricot knitted fabric (thickness: approx. 250 μm) of PET spun yarn (count: equivalent to approx. 50 D) in the same manner as in Example 1. On the spreading layer was coated the spreading action-controlling coating solution having the below-stated composition in the amount of 185 ml per 1 m². The coated layer was dried to give an integral multilayer analytical element (Example 4).

Spreading action-controlling coating solution

| | |
|---|---|
| Methylcellulose (viscosity 50 cps at 20°C, measured on 2% aqueous solution) | 20 g |
| Titanium dioxide microparticles (the same kind as in Example 3) | 150 g |
| Octylphenoxy polyethoxyethanol | 5.85 g |
| Water | 1000 ml |

Separately, an integral multilayer analytical element was prepared in the same manner as above (Example 4) except that the porous spreading layer was produced using a plain cloth of spun yarn (mixture of PET and cotton, 35/65, approx. 60 S) in place of the tricot knitted fabric and was not coated with the spreading action-controlling coating solution (Comparison Example 4).

Each of the multilayer analytical element was cut into a square chip (15 mm×15 mm) and encased into a plastic frame in the type illustrated in Fig. 4 of Japanese Patent Provisional Publication No. 57 (1982)-63452 to give an analytical slide.

Each of human blood samples having different hematocrit values indicated in Table 5 (in which the sample showing a hematocrit value of 0 was a plasma) was spotted on the porous spreading layer of the analytical slide, and the spread area was measured. The results are set forth in Table 5.

9

TABLE 5

| Spotted amount of whole blood | Example 4 10 µl | Comparison Example 4 | |
| --- | --- | --- | --- |
| | | 6 µl | 10 µl |
| Hematocrit value 0 | 92 mm² | 150 mm² | * |
| 10 | 83 mm² | 100 mm² | * |
| 40 | 70 mm² | 79 mm² | * |
| 59 | 59 mm² | 69 mm² | * |
| 58 | 53 mm² | 69 mm² | * |

Remark
*: The spread area exceeded the periphery of the square chip (15×15 mm), and accordingly no practical use in quantitative analysis was expected.

The results given in Table 5 teach that the relationship between the hematocrit value (y%) and the spread area (x mm²) is expressed by the following linear equation:

$$y = -0.5642x + 91.93$$

over the hematocrit value region of whole blood sample of from 0 (namely, plasma) to at least 68 in the case of using the integral multilayer analytical element (Example 4). The correlation coefficient (r) is −0.9994s. In contrast, while the relationship between the hematocrit value and the spread area can be expressed by a linear equation within the hematocrit value region of from approx. 15 to at least 68 in the conventional integral multilayer analytical element (Comparison Example 4), the spread area remarkably decreases as the hematocrit value increases within the hematocrit value region of from 0 (plasma) to approx. 15. Accordingly, in the latter region (0 to approx. 15), no linear equation is given.

Accordingly it has been confirmed that a hematocrit value of a generally employed whole blood sample can be determined by the measurement of the spread area by the use of the integral multilayer analytical element of the invention which has a knitted fabric spreading layer. Moreover, the relationship between a value measured on a plasma or serum and a value measured on a whole blood having an ordinary hematocrit value are easily and accurately determined and the conversion therebetween can be easily and accurately done.

Example 5
An aqueous gelatin solution having the below-stated composition was coated on a gelatin subbing layer of a colorless, transparent polyethylene terephthalate (PET) film (support, thickness: 180 µm) to give a layer of thickness of 10 µm (dry basis). The coated layer was then dried to give a water-absorbing layer.

Aqueous gelatin solution for forming water-absorbing layer

| | |
| --- | --- |
| Deionized gelatin | 10 g |
| Polyoxyethylene nonylphenyl ether (containing 10(average) oxyethylene units) | 0.01 g |
| Water | 90 g |

The water-absorbing layer was wetted uniformly with water in the amount 30 g/m² and provided thereon with a spreading layer of a tricot knitted fabric (thickness: approx. 250 µm, 36 gauge) of alkali-treated PET spun yarn (count: equivalent to approx. 50 D) under light pressure. Thus, an integral multilayer analytical element having a knitted spreading layer was prepared.

On the knitted fabric spreading layer of the multi-layer analytical element was spotted a control serum (Versatol-P; tradename of Warner-Lambert Corp.) in an amount of from 2 µl to 12 µl (increase by 2 µl). The spread area was then measured on the knitted fabric spreading layer. The results are set forth in Table 6.

TABLE 6

| Spotted amount (µl) | 2 | 4 | 6 | 8 | 10 | 12 |
|---|---|---|---|---|---|---|
| Spread area (mm$^2$) | 23 | 45 | 69 | 91 | 113 | 135 |

The results given in Table 6 apparently indicate that a vary satisfactory linear relationship is seen between the liquid amount and the spread area over a wide region of variation of amount of a spotted aqueous liquid sample (control serum).

Example 6
On a PET film (support) were sequentially formed a reagent layer for glucose determination (thickness: 15 µm, dry basis), a light-shielding layer (thickness: 5 µm, dry basis) and an adhesive layer (thickness: 2 µm) in the same manner as in Example 3.
The adhesive layer was wetted uniformly with water in the amount 30 g/m$^2$ and provided thereon with a spreading layer of a tricot knitted fabric (thickness: approx. 250 µm, 36 gauge) of alkali-treated PET spun yarn (count: equivalent to approx. 50 D) under light pressure. Thus, an integral multilayer analytical element for quantitative determination of glucose having a knitted spreading layer was prepared.
On the knitted fabric spreading of the multilayer analytical element was spotted 10 µl of the same human serum as in Example 3. The glucose concentration in the serum was then determined in the same manner as in Example 3 to give almost same results. The spread area of 10 µl of the human serum on the spreading layer was approx. 110 mm$^2$.

**Claims**

1. An integral multilayer analytical element for the analysis of an analyte in a liquid sample having, in order,
   a porous spreading layer;
   a water-absorbing or water-permeable layer containing a hydrophilic polymer which may contain at least one reagent being reactive to the analyte in the liquid sample to show a detectable change; and
   a light-transmissive, water-impermeable support;
   characterized in that said porous spreading layer is made of knitted fabric capable of spreading the liquid sample applied thereonto in a lateral direction so as to supply the liquid sample into the water-absorbing or water-permeable layer, at a substantially uniform volume per unit surface area.
2. The integral multilayer analytical element as claimed in claim 1, wherein the porous spreading layer contains at least one reagent which is reactive to the analyte in the liquid sample to show a detectable change.
3. The integral multilayer analytical element as claimed in claim 1, wherein the knitted fabric is warp knitting.
4. The integral multilayer analytical element as claimed in claim 1, wherein the knitted fabric is weft knitting.
5. The integral multilayer analytical element as claimed in claim 1, wherein the knitted fabric is single atlas stitch fabric, a single tricot stitch fabric, a double tricot stitch fabric, a milanese stitch fabric or a raschel stitch fabric.
6. The integral multilayer analytical element as claimed in claim 1, wherein the knitted fabric is a plain stitch fabric, a pearl stitch fabric, a rib stitch fabric or an interlock stitch fabric.
7. The integral multilayer analytical element as claimed in claim 1, wherein the knitted fabric is a defatted fabric.
8. Use of the integral multilayer analytical element as claimed in any of claims 1 to 7 for determination of a hematocrit value or a hemoglobin concentration in a whole blood sample.

**Patentansprüche**

1. Integrales analytisches Vielschichtelement zur Analyse einer zu bestimmenden Substanz in einer Flüssigkeitsprobe, welches der Reihe nach
   eine poröse Ausbreitungsschicht;
   eine ein hydrophiles Polymeres enthaltende wasserabsorbierende oder wasserdurchlässige Schicht, die mindestens ein Reagens enthalten kann, das gegenüber der zu bestimmenden Substanz in der Flüssigkeitsprobe reaktiv ist, um eine erfaßbare Veränderung zu zeigen; und
   einen lichtdurchlässigen, wasserundurchlässigen Träger
   aufweist, dadurch gekennzeichnet, daß die poröse Ausbreitungsschicht aus einem gestrickten bzw. gewirkten Stoff hergestellt ist, der dazu imstande ist, die darauf aufgebrachte Flüssigkeitsprobe in seitlicher Richtung auszubreiten, so daß die Flüssigkeitsprobe in die wasserabsorbierende oder wasserpermeable Schicht mit im wesentlichen gleichförmigem Volumen pro Oberflächeneinheit hineingeführt wird.

11

2. Integrales analytisches Vielschichtelement nach Anspruch 1, dadurch gekennzeichnet, daß die poröse Ausbreitungsschicht mindestens ein Reagens enthält, das gegenüber der zu bestimmenden Substanz in der Flüssigkeitsprobe reaktiv ist, um eine erfaßbare Veränderung zu zeigen.

3. Integrales analytisches Vielschichtelement nach Anspruch 1, dadurch gekennzeichnet, daß der gestrickte bzw. gewirkte Stoff durch Kettenwirken hergestellt ist.

4. Integrales analytisches Vielschichtelement nach Anspruch 1, dadurch gekennzeichnet, daß der gestrickte bzw. gewirkte Stoff durch Schußwirken hergestellt ist.

5. Integrales analytisches Vielschichtelement nach Anspruch 1, dadurch gekennzeichnet, daß der gestrickte bzw. gewirkte Stoff ein Einzel-Atlasmaschenstoff, ein Einzel-Trikotmaschenstoff, ein Doppel-Trikotmaschenstoff, ein Mailänder-Maschenstoff oder ein Raschel-Maschenstoff ist.

6. Integrales analytisches Vielschichtelement nach Anspruch 1, dadurch gekennzeichnet, daß der gestrickte bzw. gewirkte Stoff ein glatter Maschenstoff, ein Perl-Maschenstoff, ein Rippen-Maschenstoff oder ein Interlock-Maschenstoff ist.

7. Integrales analytisches Vielschichtelement nach Anspruch 1, dadurch gekennzeichnet, daß der gestrickte bzw. gewirkte Stoff ein entfetteter Stoff ist.

8. Verwendung des integralen analytischen Vielshichtelementes nach einem der Ansprüche 1 bis 7 zur Bestimmung eines Hämatokritwerts oder einer Hämoglobinkonzentration in einer Gesamtblutprobe.

**Revendications**

1. Elément intégral d'analyse à plusieurs couches pour l'analyse d'un analyte dans un échantillon liquide comportant, dans l'ordre,
une couche d'étalement poreuse;
une couche absorbant l'eau ou perméable à l'eau, contenant un polymère hydrophile qui peut renfermer au moins un réactif apte à réagir avec l'analyte de l'échantillon liquide en manifestant un changement décelable; et
un support transparent à la lumière et imperméable à l'eau;
caractérisé en ce que ladite couche d'étalement poreuse est constituée en un tissu tricoté apte à étaler l'échantillon liquide, appliqué sur celui-ci, dans une direction latérale de manière à alimenter en échantillon liquide la couche absorbant l'eau ou perméable à l'eau, en un volume sensiblement uniforme par unité de surface.

2. Elément intégral d'analyse à plusieurs couches selon la revendication 1, dans lequel la couche d'étalement poreuse contient au moins un réactif qui est apte à réagir avec l'analyte de l'échantillon liquide en manifestant un changement décelable.

3. Elément intégral d'analyse à plusieurs couches selon la revendication 1, dans lequel le tissu tricoté est un article de tricotage-chaîne.

4. Elément intégral d'analyse à plusieurs couches selon la revendication 1, dans lequel le tissu tricoté est un article de tricotage-trame.

5. Elément intégral d'analyse à plusieurs couches selon la revendication 1, dans lequel le tissu tricoté est un tissu à maille satin simple, un tissu à maille tricot simple, un tissu à maille tricot double, un tissu à maille milanaise ou un tissu à maille Rachel.

6. Elément intégral d'analyse à plusieurs couches selon la revendication 1, dans lequel le tissu tricoté est un tissu à maille à l'endroit, un tissu à maille perlée, un tissu à maille à côtes ou un tissu à maille interlock.

7. Elément intégral d'analyse à plusieurs couches selon la revendication 1, dans lequel le tissu tricoté est un tissu dégraissé.

8. Utilisation de l'élément intégral d'analyse à plusieurs couches selon l'une quelconque des revendications 1 à 7 pour la détermination de la valeur de l'hématocrite ou de la concentration d'hémoglobine d'un échantillon de sang complet.